# EUROPEAN PATENT APPLICATION

(11) **EP 1 028 141 A1**
(43) Date of publication of application: **16.08.2000**
(21) Application number: 00102578.2
(22) Date of filing: 10.02.2000
(51) Int. Cl.: C08K 5/098, C08K 5/20, C08L 25/10, A61M 5/315

(54) **Medical article of improved stick-slip**

(30) Priority: 12.02.1999 US 249928
(71) Applicant: Becton, Dickinson and Company, Franklin Lakes, New Jersey 07417-1880 (US)
(72) Inventor: Liebmann-Vinson, Andrea, Willow Springs, North Carolina 27592 (US); Knors, Christopher J., Raleigh, North Carolina 27612 (US); Ghosh, Jayanto K., Little Falls, New Jersey 07424 (US); Nelson, Linda H., Ridgewood, New Jersey 07450 (US); Kaduk, Bruce A., Montville, New Jersey 07045 (US)
(74) Representative: von Kreisler, Alek, Dipl.-Chem.

(57) **Abstract**

A composition comprising a styrene-butadiene-styrene or styrene-ethylene-butylene-styrene copolymer, an aromatic polyether, a polyolefin, a particular wax and a plasticizing oil is molded to give a medical article. A syringe assembly includes a barrel and a stopper of the composition mounted on a plunger rod. The syringe assembly operates with minimal stick-slip. The invention includes a method to use the syringe assembly.

## Description

1. Field of the Invention: This invention relates to medical articles, and more particularly relates to medical articles including sliding surfaces of improved stick-slip.

2. Background: Medical articles are conventionally made by thermoplastic processing of polyolefins or copolymers thereof. Typical plastics used are polyethylene, polypropylene or copolymers of these materials with one or more other monomers such as styrene, acrylic or butadiene. A variety of physical properties can be achieved according to the medical application envisioned for the plastic.

One important criterion to be considered when selecting a plastic for medical application is the morphological stability of the plastic. Thus, the plastic must not undergo any substantial change in size or shape during prolonged storage which would compromise the specifications required for proper end use. This is particularly true for multicomponent articles having contiguous parts of close tolerances where avoidance of size changes is very important if sealing contact between the parts must be maintained.

If the sealing components also have a sliding relationship, the phenomenon known as stick-slip is often a problem. Thus, it is well known that two plastic surfaces having a sliding relationship often exhibit sufficient resistance to initiation of sliding movement that gradually increased pressure applied to one of the surfaces does not cause movement until a threshold pressure is reached at which point a sudden sliding separation of the surfaces takes place. This situation is commonly referred to as stick-slip or sticktion. Stick-slip is exacerbated by prolonged stationary contact between the surfaces, such as occurs during shelf time. It is particularly troublesome in devices such as syringes, in particular syringes to be used with syringe pumps where ultra slow advancement of the stopper is required. The term tribology refers to the study of friction, lubrication and wear of surfaces in relative motion.

Natural rubber has been compression molded into articles of low stick-slip by complex manufacturing processes including long cycle times. A variety of thermoplastic elastomers has also been used. Such materials however are well known to exhibit substantial stick-slip, and their use requires additives to minimize the stick-slip.

It is toward provision of multicomponent medical articles of improved seal retention and minimal stick-slip that the present invention is directed.

### Summary of the Invention

A medical article is molded from a composition which includes a styrene-butadiene-styrene copolymer, preferably a styrene-ethylene-butylene-styrene, and a particular wax. Preferred compositions also include a polyolefin, an aromatic polyether, and a plasticizer. Preferred articles are multicomponent articles in which two components, at least one of which is of the aforementioned composition, are in a sealing and sliding relationship. In accordance with the invention, such articles exhibit minimal stick-slip. The preferred article is a syringe having a stopper which slides in a barrel wherein the stopper is of the disclosed composition.

### Brief Description of the Drawings

Fig. 1 is a perspective view of a typical syringe barrel as used in the syringe assembly of the invention;
Figs. 2 and 3 are perspective views of syringe stoppers of the invention;
Fig. 4 is a plot illustrating the presence of stick-slip for syringe assembly including the barrel of Fig. 1 and a stopper of a conventional prior art thermoplastic elastomer;
Fig. 5 is a plot showing absence of stick-slip for a syringe assembly including the barrel of Fig. 1 and a stopper of natural rubber;
Figs. 6 and 7 are plots illustrating absence of stick-slip for a syringe assembly including a stopper of the composition of the invention; and
Figs. 8 and 9 are plots showing the presence of stick-slip for a syringe assembly including stoppers of the compositions of Figs. 6 and 7 but lacking the wax.

### Detailed Description

While this invention is satisfied by embodiments in many different forms, there will herein be described in detail embodiments of the invention with the understanding that the present disclosure is to be considered as exemplary of the principles of the invention and is not intended to limit the invention to the embodiments illustrated and described. The scope of the invention will be measured by the appended claims and their equivalents.

The medical article contemplated by the invention prepared from the disclosed composition may be, for example, a needle shield, tubing, seal, gasket, syringe stopper or tube stopper. Preferred medical articles are multicomponent articles where close tolerances must be maintained during end use. The most preferred medical article is a syringe assembly, either 2-piece or 3-piece, in which the sliding seal between the stopper and barrel remains uncompromised during long term storage and exhibits minimal stick-slip. The invention will henceforth be described for the syringe assembly with the understanding that the description thereof may equally well be applied to other medical articles which would benefit from the outstanding sealability and minimal stick-slip provided by the composition disclosed.

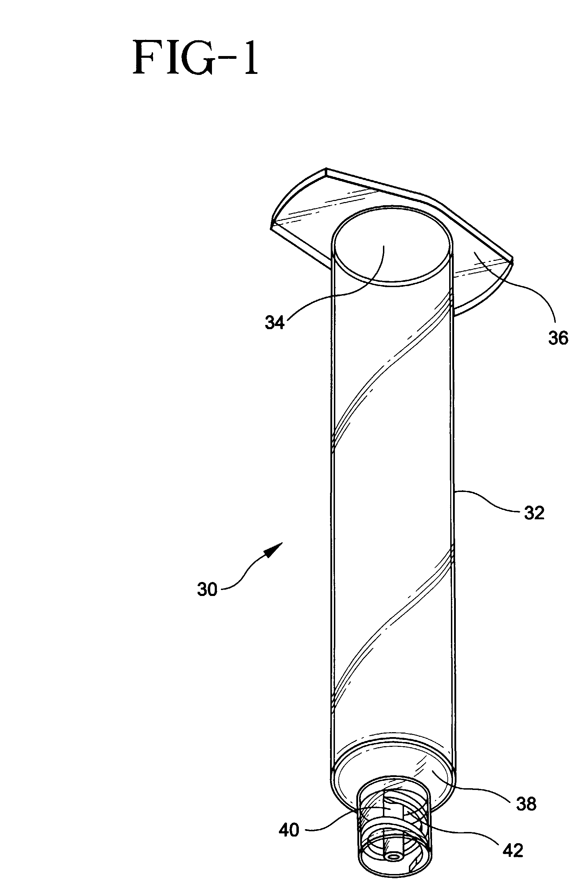

In Fig 1, a syringe barrel 30 has a body portion 32 having an open top end 34 and an optional grasping portion 36. A bottom wall 38 includes a tube portion 40 for delivery of a fluid from body portion 32 through a needle (not shown in the drawing) when the assembly is in use. Bottom portion 38 is affixed to a conventional hub 42.

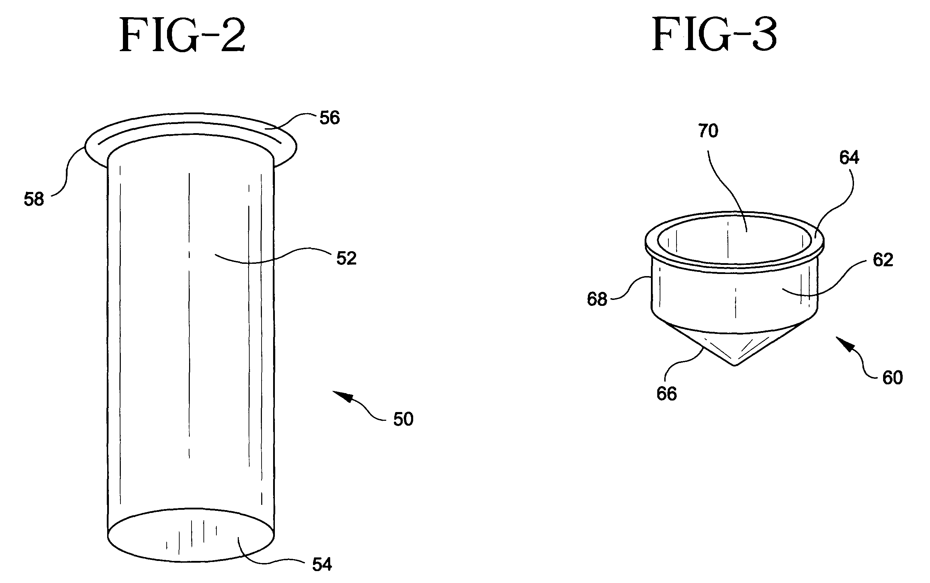

In Fig. 2, syringe stopper 50 has a body portion 52 and a bottom wall 54. A top wall 56 has an annular projection 58 for advancing and retracting the stopper in the barrel when the assembly is in use. Stopper 50 is dimensioned to have a sliding and sealing relationship to body portion 32.

Fig. 3 illustrates a syringe stopper adapted for use with a conventional plunger rod (not shown) in a 3-piece syringe assembly. A stopper 60 has a body portion 62, a top wall 64, a bottom wall 66 tapering to a point and a side wall 68. Body portion 62 has a recess 70 into which the plunger rod is securely mounted. The rod-stopper assembly is inserted into the barrel so that the stopper slides sealingly against the barrel inside wall. Although not shown in the drawings, a conventional lubricant, such as polydimethyl siloxane, may be positioned between the barrel and stopper.

A first component of the composition of the invention may be a styrene-butadiene-styrene (SBS) copolymer. Preferred copolymers are styrene-ethylene-butylene-styrene (SEBS) copolymers. Copolymers of SBS and SEBS are well known in the art and are commercially available from Shell (Houston, Texas) under the name KRATON™. In preferred copolymers of the invention about 40-100% of the olefinic linkages are saturated.

An optional second component of the preferred composition is an aromatic polyether. The preferred aromatic polyether is a polyphenylene oxide (PPO), most preferably a polyether derived from 2,6-dimethylphenol having an intrinsic viscosity of 0.15 to 0.51, preferably 0.3 to 0.46 dl/g.

The preferred composition also includes an optional component, such as a polyolefin and a plasticizing oil, which contribute to melt processing. The polyolefin may have 2-8, preferably 3-6 carbon atoms in the repeating unit, and most preferably is polypropylene. Any plasticizing oil as known in the art may be used, preferably a mineral oil. Selection of suitable processing components is well within the purview of one skilled in the art, and no further description of these components of the composition is necessary for a full understanding of this invention..

In accordance with the invention, it has been found that stick-slip is substantially improved or eliminated if the composition includes a particular wax. Preferred waxes are salts or amides of long chain fatty acids having 6 to 36, preferably 12-30 carbon atoms. The most preferred waxes are amides, such as stearamide and erucamide, and stearate salts, such as alkali metal, ammonium, aluminum, calcium or zinc stearates. The stearate salt waxes are sold commercially by R.T. Vanderbilt Co. Inc., Norwalk, CT, under the trade name VANFRE™, and the amide waxes are sold by Witco Corp, Greenwich, CT., under the trade name KEMAMIDE™. The most preferred wax is a mixed-metal wax containing zinc stearate (VANFRE AP-2S).

The SBS or SEBS may be present in the preferred composition at 10-40, preferably 20-30 weight percent. The PPO may be present at 0-40, preferably 5-20 weight percent. The plasticizer may be present at 2-50, preferably 5-40 weight percent. The polyolefin may be present at 1-20, preferably about 5-10 weight percent. The wax may be present at 0.5 to 10, preferably 2.5 to 7.5 weight percent of the composition. Commercial compositions containing the SEBS, polyether, polyolefin and mineral oil are commercially available from Shell under the trade name KRATON™, from GLS Corp. (Cary, Il) under the trade name DYNAFLEX™ and from Teknor-Apex, (Pawtucket, RI). These commercial products may be blended with the wax to give preferred compositions of the invention.

Other additives as known in the art may be added to the composition to provide other desirable properties. For example, fillers, preferably inorganic compounds such as calcium carbonate, coloring agents, radiation stabilizers, antistatic materials, wetting agents, foaming agents, thermal oxidative stabilizers and the like may be added in suitable quantities. Such additives may be present at 0-40 weight percent of the composition. One skilled in the art of polymer compositions is well versed in such additives and no further description of these materials is needed for a complete understanding of the invention.

The wax and other additives may be blended with the SEBS in any sequence to give the composition of the invention by any conventional mixing procedure using standard blending equipment, such as a Banbury mixer or a twin screw extruder.

For the syringe of the invention, the barrel in which the stopper of the composition slides may be of glass or any suitable plastic, such as polypropylene. A preferred syringe barrel is molded of metallocene cyclic olefin copolymer (COC). These products are well known in the art and are commercially available. Representative COC trade names are TOPAS™ (Hoechst Advanced Technology Group, Summit, NJ, ZEONEX™ (Nippon Zeno Co.) and APEL™ (Mitsui Petrochemical Industries). Most preferably, the barrel of polypropylene or COC contains an external barrier coating, such as for example a siliceous material. A preferred coating material is a silicon oxide. COC copolymers, as well as molding and coating on syringe barrels, are well known in the art, and no further details of this aspect of the invention are needed for a complete description and understanding thereof by one skilled in the art.

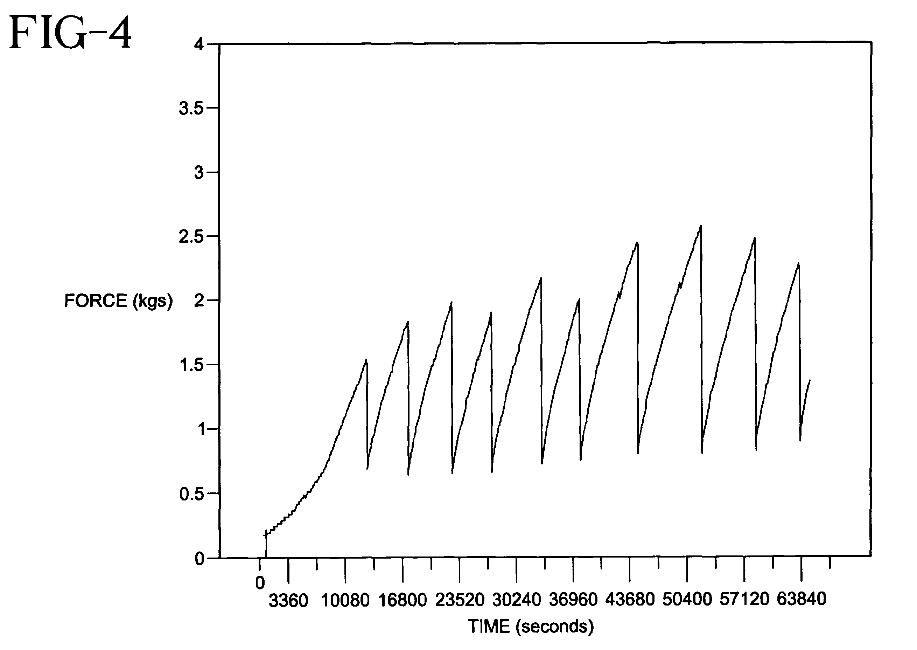

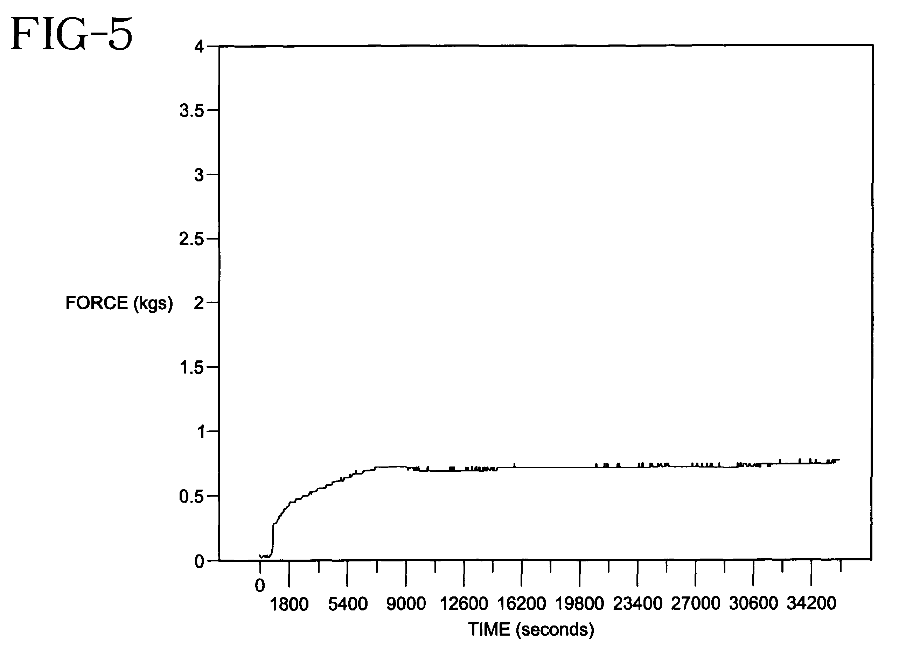

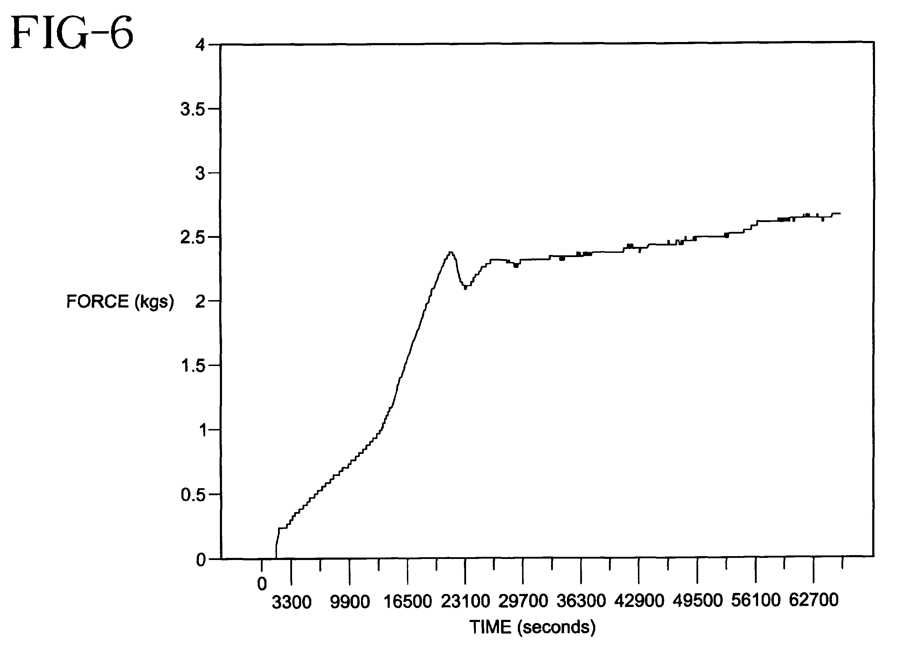

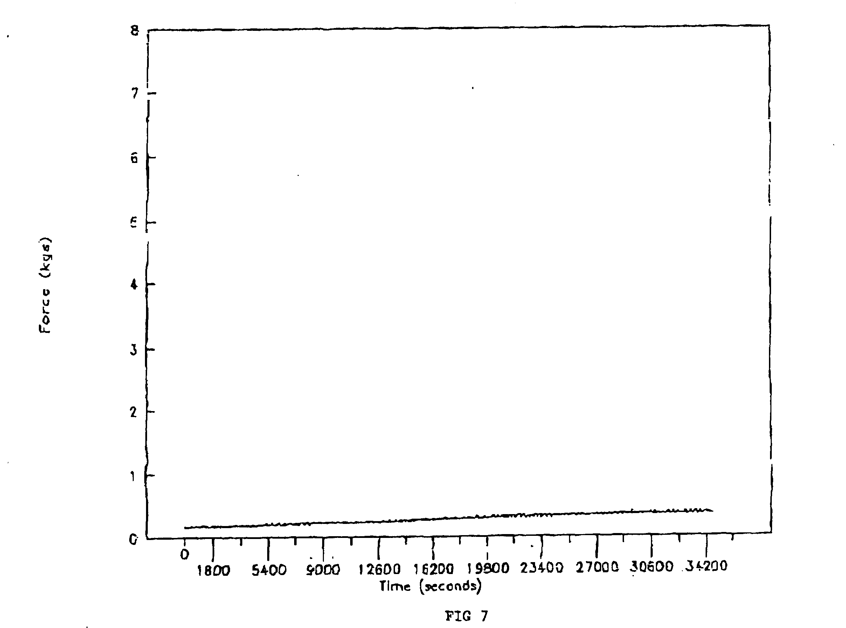

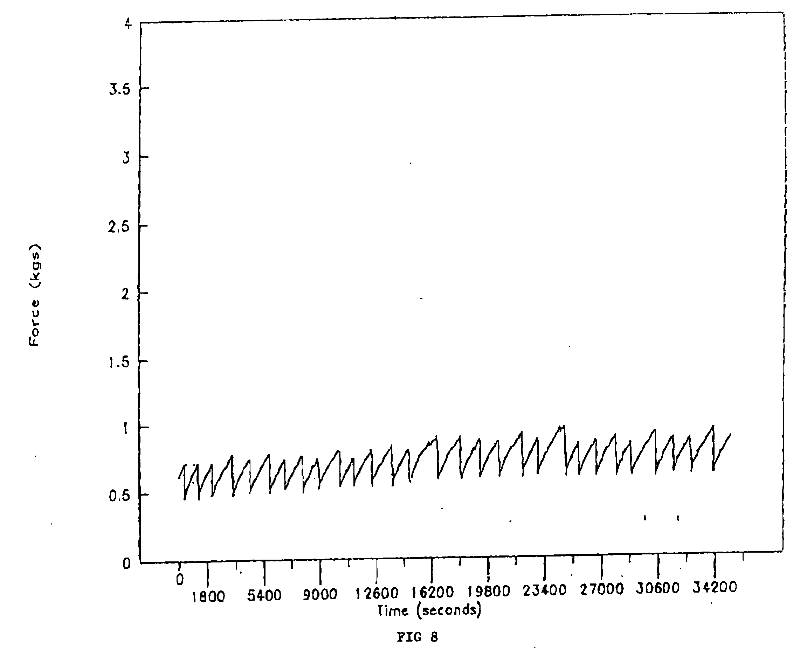

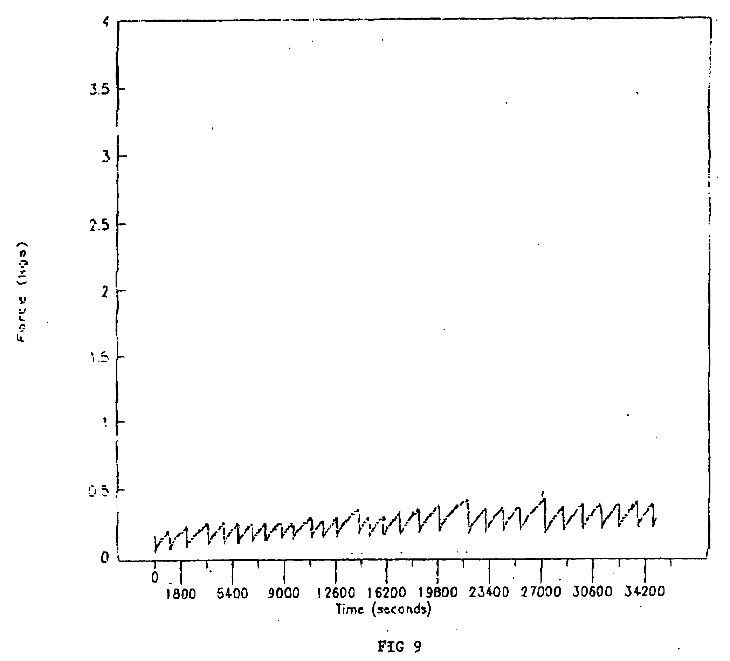

The presence or absence of stick-slip is shown by plots of time vs. force required to advance syringe stoppers in 3-piece 3cc and 60cc syringes mounted on syringe pumps. Figs. 4 and 5 are control experiments showing stick-slip for a SANTOPRENE™ (blend of PP and ethylene-propylene-diene monomer) stopper and lack of stick-slip for a natural rubber stopper during advancement in a polypropylene barrel. Figs. 6 and 7 show no stick-slip for 60cc syringes including stoppers of the composition of the invention. Figs. 8 and 9 show that in absence of the wax in the stoppers, stick-slip is present in the syringes of Figs. 6 and 7.

### Experimental

### Example 1 - Measurement of Stick-slip

A syringe having a polypropylene barrel and plunger rod and stoppers of Example 2 was mounted onto a syringe pump fitted with a force transducer which grips the thumbpress of the stopper. The transducer was calibrated to the "0" line on the monitor of a computerized data acquisition system and the syringe barrel filled with deionized water and purged of air bubbles. The pump was set for the slowest possible speed (0.1ml/hr) to give maximum stick-slip. Force data against time was collected at the monitor.

### Example 2 - Invention

The components described above were blended into compositions having varying quantities of wax as shown in the Table. The compositions were molded into syringe stoppers. The stoppers were affixed onto the plunger rod of a conventional 3-piece syringe having a polypropylene barrel and plunger and the syringe was mounted onto a conventional syringe pump. The force required to advance the plunger rod at a volumetric flow rate of 0.1 ml/hr was applied and stick-slip was determined in accordance with Example 1.

**TABLE**

| Composition | Syringe Size (cc) | SEBS | Wax-% | Force kg. | Stick-slip |
|---|---|---|---|---|---|
| 1 | 60 | a | - | 0.71 | y ...(Fig 8) |
| 2 | 3 | a | - | 0.59 | y |
| 3 | 60 | b | - | 1.00;0.68 | y |
| 4 | 3 | b | - | 0.54 | y |
| 5 | 60 | a | c-2.5 | 0.89;0.48 | n |
| 6 | 60 | a | c.5.0 | 0.32;0.52 | n ...(Fig.6) |
| 7 | 60 | a | c-10.0 | 0.07;0.36 | n |
| 8 | 60 | a | d-2.5 | 0.01 | n |
| 9 | 3 | b | d-2.5 | 0.22 | n |
| 10 | 3 | b | e-2.5 | 0.00 | n |
| 11 | 3 | b | e-5.0 | 0.08 | n |
| 12 | 60 | b | c-2.5 | 0.46 | n-y |
| 13 | 60 | b | c-5.0 | 0.32 | n |
| 14 | 60 | b | c-10.0 | 0.07 | n |
| 15 | 60 | b | d-2.5 | 0.25 | n |
| 16 | 60 | b | d-5.0 | 0.21 | n |
| 17 | 60 | a | c-0.5 | 0.92 | y |
| 18 | 60 | a | c-1.0 | 1.23 | y |
| 19 | 60 | a | c-1.5 | 0.94 | y |
| 20 | 60 | a | c-2.0 | 0.91 | y |
| 21 | 60 | a | f-2.5 | 0.74 | y |
| 22 | 60 | a | f-10.0 | 0.78 | y |
| 23 | 60 | a | g-2.5 | 0.48 | y |
| 24 | 60 | a | g-10.0 | 0.42 | y |
| 25 | 60 | a | h-2.5 | 1.00 | y |
| 26 | 60 | a | i-2.5 | 1.34 | y |
| 27 | 60 | j | - | 0.4 | y ...(Fig. 9) |
| 28 | 60 | j | c-5.0 | 0.4 | n ...(Fig. 7) |
| a....Dynaflex 7722 b....Dynaflex LC-142-096 c....Vanfre AP-2S Fatty acid salt wax d....Kemamide S-221 fatty acid amide wax e...Kemamide -ULTRA f....AC-629 polyethylene wax (Allied-Signal Corp) g...AC-656 polyethylene wax (Allied-Signal Corp) h...MB-50-001 silicone wax (Dow Corning) i....MB-50-002 silicone wax (Dow Corning) j...SBS-SEBS (90:10 mixture by wt.), mineral oil, polypropylene | | | | | |

In the Tables, the presence of stick-slip is shown by y, the absence of stick-slip by n. It is seen from compositions 1-4 that SEBS, in absence of the wax, exhibits stick-slip. Compositions 5-16 of the invention show the absence of stick-slip in both 3 and 60cc syringes with stoppers of two commercial DYNAFLEX compositions including 3 different VANFRE and KEMAMIDE waxes at different concentrations. Comparative compositions 17-26 show the presence of stick-slip in DYNAFLEX compositions having lower concentrations of VANFRE and several concentrations of commercially available silicone and polyethylene waxes. Composition 28 of SBS and wax is free of stick-slip whereas composition 27 of SBS but lacking the wax shows stick-slip.

## Claims

1. A composition comprising a blend of a copolymer selected from the group consisting of a styrene-butadiene-styrene and a styrene-ethylene-butylene-styrene and a wax comprising a salt or amide of a fatty acid having at least 6 carbon atoms.

2. The article of Claim 1 further comprising an aromatic polyether.

3. The article of Claim 1 further comprising a component selected from the group consisting of a polyolefin and a plasticizer.

4. A composition comprising a blend of a styrene-ethylene-butylene-styrene copolymer, a polyolefin, a plasticizer, an aromatic polyether and a wax comprising a salt or amide of a fatty acid having from 12-30 carbon atoms.

5. A medical article comprising the composition of Claim 1.

6. A multi-component medical article comprising a first component fabricated from the composition of Claim 4.

7. The article of Claim 6 which is a syringe assembly comprising a stopper portion as said first component and a barrel portion for receiving said stopper portion in sealing and sliding engagement, said stopper portion being secured to a plunger rod.

8. The assembly of Claim 7 wherein said barrel portion is of polypropylene or a cyclic olefin copolymer.

9. The assembly of Claim 7 further comprising a barrier coating of a siliceous material on said barrel.

10. A method to administer a medicament to a patient comprising:
a) filling the barrel portion of the assembly of Claim 7 with a medicament;
b) inserting a needle portion of said assembly into the vein of a patient; and
c) applying force to said rod to cause said medicament to be discharged from said assembly.
